# EUROPEAN PATENT SPECIFICATION

(11) **EP 2 298 197 B1**
(45) Date of publication and mention of the grant of the patent: **27.01.2021**
(21) Application number: 10197008.5
(22) Date of filing: 16.06.2004
(51) Int. Cl.: A61B 17/32, A61B 17/00

(54) **Hand activated ultrasonic instrument**
Von Hand aktiviertes Ultraschallinstrument
Instrument ultrasonique activé à la main

(30) Priority: 17.06.2003 US 478984 P
(43) Date of publication of application: 23.03.2011
(62) Divisional of application: 04755466.2
(73) Proprietor: Ethicon LLC, 00969 Guaynabo (PR)
(72) Inventor: MUIR, Stephanie J., Loveland, OH 45140 (US); PROCH, Francis S., Pickerington, OH 43147 (US); DUTTA, Sudeep N., Mainneville, OH 45039 (US); NSUSER, Kenneth S., Loveland, OH 45140 (US)
(74) Representative: Tunstall, Christopher Stephen

(56) References cited:
- EP-A2- 1 199 040
- US-A- 5 873 873
- US-A- 5 938 633
- US-A- 5 989 274
- US-A- 6 056 735
- US-A1- 2002 057 541
- US-A1- 2002 107 538
- US-A1- 2003 055 417
- US-B1- 6 325 795

## Description

### Field of the Invention

The present invention relates generally to ultrasonic surgical devices, and more particularly to an ultrasonic surgical clamp coagulator apparatus for coagulating and/or cutting tissue, including a hand activated switch positioned on the handle for easy access by the surgeon.

### Background of the Invention

Ultrasonic surgical instruments are finding increasingly widespread applications in surgical procedures by virtue of the unique performance characteristics of such instruments. Depending upon specific instrument configurations and operational parameters, ultrasonic surgical instruments can provide substantially simultaneous cutting of tissue and hemostasis by coagulation, desirably minimizing patient trauma. The cutting action is typically effected by an end-effector at the distal end of the instrument, with the end-effector transmitting ultrasonic energy to tissue brought into contact therewith. Ultrasonic instruments of this nature can be configured for open surgical use, or laparoscopic or endoscopic surgical procedures.

Ultrasonic surgical instruments have been developed that include a clamp mechanism to press tissue against the end-effector (i.e. the cutting blade) of the instrument in order to couple ultrasonic energy to the tissue of a patient. Such an arrangement (sometimes referred to as a clamp coagulator shears or an ultrasonic transactor) is disclosed in U.S. Pat. Nos. 5,322,055; 5,873,873 and 6,325,811. The surgeon activates the clamp pad to press against the end-effector by squeezing on the handgrip or handle.

A foot pedal operated by the surgeon while simultaneously applying pressure to the handle to press tissue between the clamp pad and end-effector activates a generator that provides energy that is transmitted to the cutting blade for cutting and coagulating tissue. Key drawbacks with this type of instrument activation include the loss of focus on the surgical field while the surgeon searches for the foot pedal, the foot pedal getting in the way of the surgeon's movement during a procedure and surgeon leg fatigue during long cases.

US-A-6056735 discloses various embodiments of an ultrasound treatment system. The systems include a handpiece having an ultrasonic transducer serving as a vibration conveyor for stationary section of a treatment unit used to treat a living tissue. A manipulator is provided for handling or clamping or freeing a living tissue. One embodiment provides a switch for activating the transducer on the manipulator.

US-A-2003/0055417 discloses a surgical system for controlled application of ultrasound energy to engaged tissue volumes.

US-A-5873873 discloses an ultrasonic clamp coagulator apparatus adapted to effect cutting, coagulation, and clamping of tissue by cooperation of a clamping mechanism of the apparatus with an associated ultrasonic end-effector. The apparatus comprises a handgrip portion with which a user may hold the apparatus. A triggering mechanism is provided, which preferably comprises a foot activating switch. US-A-5938633 discloses a similar triggering mechanism used for a different surgical device.

US-A-2002/0057541 discloses a surgical handpiece system comprising a switch assembly mounted on a handpiece body. Various circuit configurations are disclosed, including an arrangement which permits a switch end cap, which is attached to the handpiece, to be freely rotated about the handpiece body.

US-A-2002/0107538 discloses various different ultrasonic operation systems, including a device having a scissors type handpiece. A handpiece component comprises switches may be mounted on the handpiece plug of this device.

US-A-5989274 illustrates an ultrasonic instrument comprising a pushbutton located on a finger grip of the handle. The pushbutton is an alternative to the foot activating switch which may activate a generator to drive the instrument.

The present invention overcomes the drawbacks of the prior art and is directed to an improved ultrasonic surgical clamp coagulator shears apparatus that provides for a more ergonomic means for activating the shears by incorporating fingertip control on the handles.

### Brief Summary of the Invention

The present invention provides an utrasonic surgical instrument according to the claims.

### Brief Description of the Figures

FIGURE 1 is a perspective view of an ultrasonic surgical system including an ultrasonic clamp coagulator apparatus embodying the principles of the present invention;
FIGURE 2 is an enlarged, elevation view fragmentary perspective view of a clamp mechanism of the clamp coagulator apparatus of Fig. 1;
FIGURE 3 is a side elevation, partially cut-away view, of the clamp coagulator embodying the principles of the present invention;
FIGURE 4 is an assembly drawing of a clamp coagulator of the present invention;
FIGURE 5 is an exploded view of the handle incorporating the rocker switch, handpiece connector, two slip rings and flex circuit;
FIGURES 6a-b are exploded views of the large slip ring and small slip ring, respectively;
FIGURE 7 is an exploded view of the handpiece connector;
FIGURE 8a-b is an exploded view of the flex circuit apparatus and the associated electrical schematic, respectively;
FIGURE 9 is a schematic view of the handle of an ultrasonic instrument of the present invention illustrating dimensional placement of the switches; and FIGURE 10 is an exploded view of the switch assembly.

In this specifications the following non-SI units are used, which may be converted to the respective SI or metric unit according to the following conversion table:

**SI unit conversion table**

| *Name of unit* | *Symbol* | *Conversion factor* | *Si or metric unit* |
|---|---|---|---|
| *inch* | *inch, in,"* | *2,54* | *centimetres, cm* |

### Detailed Description of the Invention

Before explaining the present invention in detail, it should be noted that the invention is not limited in its application or use to the details of construction and arrangement of parts illustrated in the accompanying drawings and description. The illustrative embodiments of the invention may be implemented or incorporated in other embodiments, variations and modifications, and may be practiced or carried out in various ways. Furthermore, unless otherwise indicated, the terms and expressions employed herein have been chosen for the purpose of describing the illustrative embodiments of the present invention for the convenience of the reader and are not for the purpose of limiting the invention.

The present invention is particularly directed to an improved ultrasonic surgical clamp coagulator apparatus which is configured for effecting tissue cutting, coagulation, and/or clamping during surgical procedures. The present apparatus can readily be configured for use in both open surgical procedures, as well as laparoscopic or endoscopic procedures. Versatile use is facilitated by selective use of ultrasonic energy. When ultrasonic components of the apparatus are inactive, tissue can be readily gripped and manipulated, as desired, without tissue cutting or damage. When the ultrasonic components are activated, the apparatus permits tissue to be gripped for coupling with the ultrasonic energy to effect tissue coagulation, with application of increased pressure efficiently effecting tissue cutting and coagulation. If desired, ultrasonic energy can be applied to tissue without use of the clamping mechanism of the apparatus by appropriate manipulation of the ultrasonic "blade" or end-effector of the device.

As will become apparent from the following description, the present clamp coagulator apparatus is particularly configured for disposable use by virtue of its straightforward construction. As such, it is contemplated that the apparatus be used in association with an ultrasonic drive unit of a surgical system, whereby ultrasonic energy from the drive unit provides the desired ultrasonic actuation of the present clamp coagulator apparatus. It will be appreciated that a clamp coagulator apparatus embodying the principles of the present invention can be configured for non-disposable use, and non-detachably integrated with an associated ultrasonic drive unit. However, detachable connection of the present clamp coagulator apparatus with an associated ultrasonic drive unit is presently preferred for single-patient use of the apparatus.

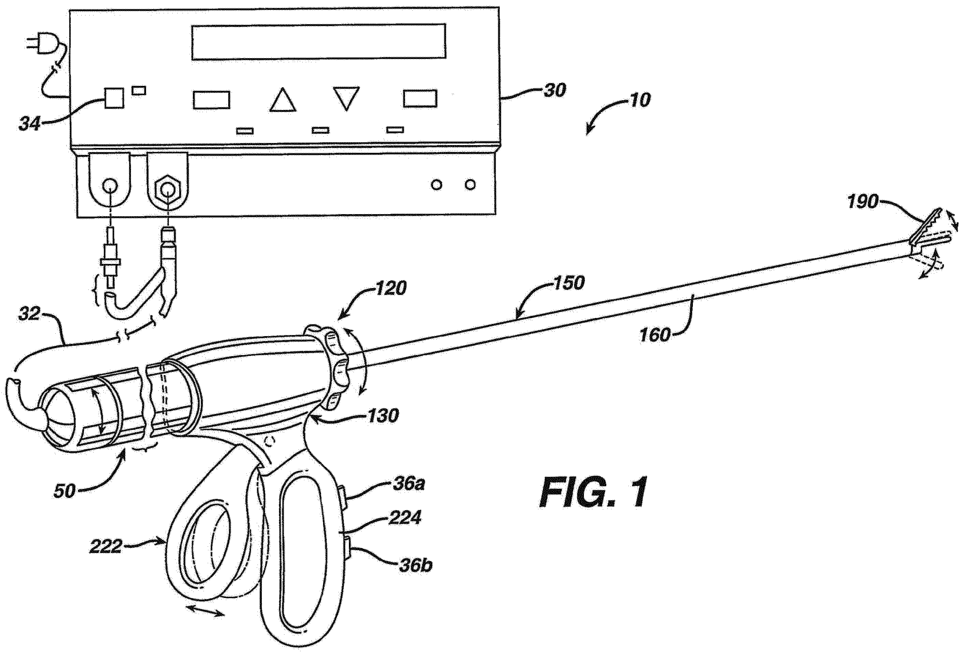

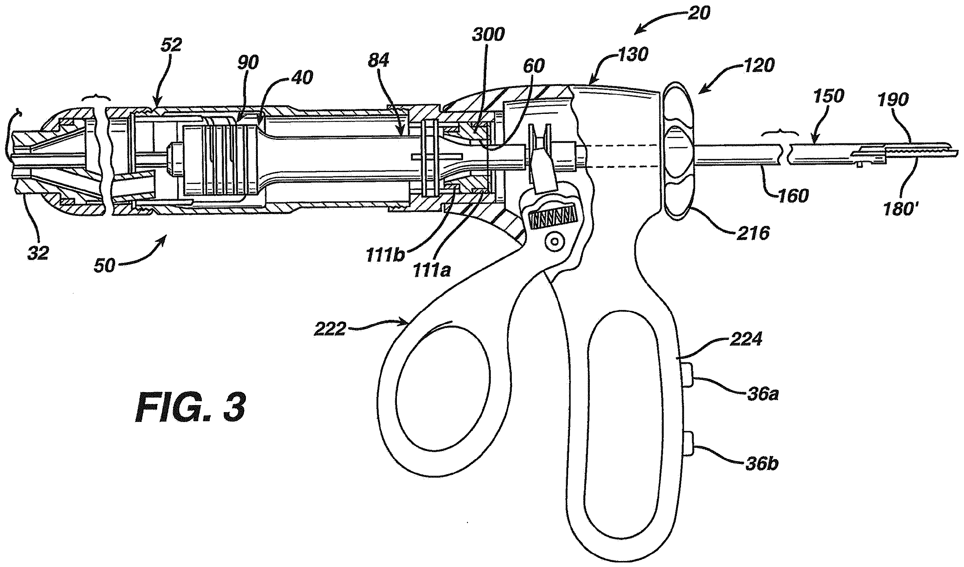

With reference first to FIGS. 1 and 3, therein is illustrated a presently preferred embodiment of a surgical system, generally designated 10, which includes an ultrasonic clamp coagulator apparatus embodying the principles of the present invention. Preferred details of the ultrasonic generator and associated ultrasonic drive unit of the surgical system 10 will first be described, with subsequent detailed description of the fingertip activation of the end-effector, embodying the principles of the present invention.

The surgical system 10 includes an ultrasonic generator 30 and an associated ultrasonic surgical instrument. The surgical instrument includes an ultrasonic drive unit, designated 50, and an ultrasonic clamp coagulator apparatus 120 embodying the principles of the present invention. As will be further described, an ultrasonic transducer of the drive unit 50, and an ultrasonic waveguide of the clamp coagulator 120, together provides an acoustic assembly of the present surgical system, with the acoustic assembly providing ultrasonic energy for surgical procedures when powered by generator 30. It will be noted that in some applications, the ultrasonic drive unit 50 is referred to as a "hand piece assembly" because the surgical instrument of the surgical system is configured such that a surgeon grasps and manipulates the ultrasonic drive unit 50 during various procedures and operations. The clamp coagulator apparatus 120 embodying the principles of the present invention preferably includes a pistol-like grip arrangement that facilitates positioning and manipulation of the instrument apart from manipulation of the ultrasonic drive unit 50.

The generator 30, for example, a Generator 300 available from Ethicon Endo-Surgery, Inc., Cincinnati, Ohio, of the surgical system sends an electrical signal through a cable 32 at a selected current, frequency, and phase determined by a control system of the generator 30. As will be further described, the signal causes one or more piezoelectric elements of the acoustic assembly of the surgical instrument to expand and contract, thereby converting the electrical energy into mechanical motion. The mechanical motion results in longitudinal waves of ultrasonic energy that propagate through the acoustic assembly in an acoustic standing wave to vibrate the acoustic assembly at a selected frequency and excursion. An end-effector at the distal end of the waveguide of the acoustic assembly is placed in contact with tissue of the patient to transfer the ultrasonic energy to the tissue. As further described below, a surgical tool, such as, a jaw or clamping mechanism, is preferably utilized to press the tissue against the end-effector.

As the end-effector couples with the tissue, thermal energy or heat is generated as a result of friction, acoustic absorption, and viscous losses within the tissue. The heat is sufficient to break protein hydrogen bonds, causing the highly structured protein (i.e., collagen and muscle protein) to denature (i.e., become less organized). As the proteins are denatured, a sticky coagulum forms to seal or coagulate small blood vessels. Deep coagulation of larger blood vessels results when the effect is prolonged.

The transfer of the ultrasonic energy to the tissue causes other effects including mechanical tearing, cutting, cavitation, cell disruption, and emulsification. The amount of cutting as well as the degree of coagulation obtained varies with the excursion of the end-effector, the frequency of vibration, the amount of pressure applied by the user, the sharpness of the end-effector, and the coupling between the end-effector and the tissue.

As illustrated in FIGs. 1 and 3, the generator 30 includes a control system integral with the generator 30 and an on-off switch 34. The power switch 34 controls the electrical power to the generator 30, and when activated by the triggering mechanism 36a-b, the generator 30 provides energy to drive the acoustic assembly 40 of the surgical system 10 to drive the end-effector at a predetermined excursion level. The generator 30 drives or excites the acoustic assembly at any suitable resonant frequency of the acoustic assembly.

When the generator 30 is activated via the triggering mechanism 36a-b, the generator 30 continuously applies electrical energy to a transducer stack 90. A phase-locked loop in the control system of the generator 30 monitors feedback from the acoustic assembly. The phase lock loop adjusts the frequency of the electrical energy sent by the generator 30 to match the resonant frequency of the selected longitudinal mode of vibration of the acoustic assembly including the tissue load. In addition, a second feedback loop in the control system maintains the electrical current supplied to the acoustic assembly at a preselected constant level in order to achieve substantially constant excursion at the end-effector of the acoustic assembly.

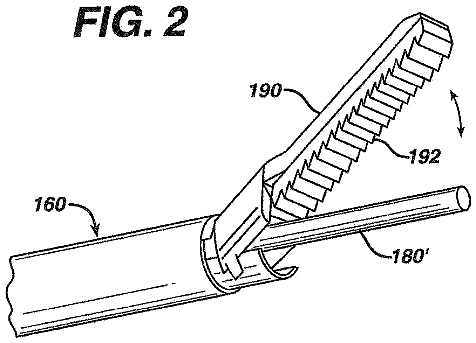

The electrical signal supplied to the acoustic assembly will cause the distal end of the waveguide, i.e., the end-effector, (FIG. 2) to vibrate longitudinally in the range of, for example, approximately 20 kHz to 250 kHz, and preferably in the range of about 54 kHz to 56 kHz, and most preferably at about 55.5 kHz. The excursion of the vibrations at the end-effector can be controlled by, for example, controlling the amplitude of the electrical signal applied to the transducer assembly 40 by the generator 30. Switch 36a provides for one level of amplitude and switch 36b provides for a second level of amplitude.

As noted above, the triggering mechanism 36a-b of the generator 30 allows a user to activate the generator 30 so that electrical energy may be continuously supplied to the acoustic assembly. The triggering mechanism 36a-b comprises a rocker switch that is positioned on handle 224 and electrically coupled or attached to the generator 30 by a cable or cord. Alternatively, in other unclaimed examples the triggering mechanism 36a-b could be placed at other convenient locations, for example, on thumb ring 222 or on shroud 130.

Referring to FIGS. 1, 3 and 4, the handpiece 50 includes a multi-piece housing 52 adapted to isolate the operator from the vibrations of the acoustic assembly. The drive unit housing 52 can be shaped to be held by a user in a conventional manner, but it is contemplated that the present clamp coagulator 120 principally be grasped and manipulated by a pistol-like arrangement provided by a housing of the apparatus, as will be described. While the multi-piece housing 52 is illustrated, the housing 52 may comprise a single or unitary component.

The housing 52 generally includes a proximal end, a distal end, and a cavity extending longitudinally therein. The distal end of the housing 52 includes an opening 60 configured to allow the acoustic assembly of the surgical system 10 to extend therethrough, and the proximal end of the housing 52 is coupled to the generator 30 by the cable 32.

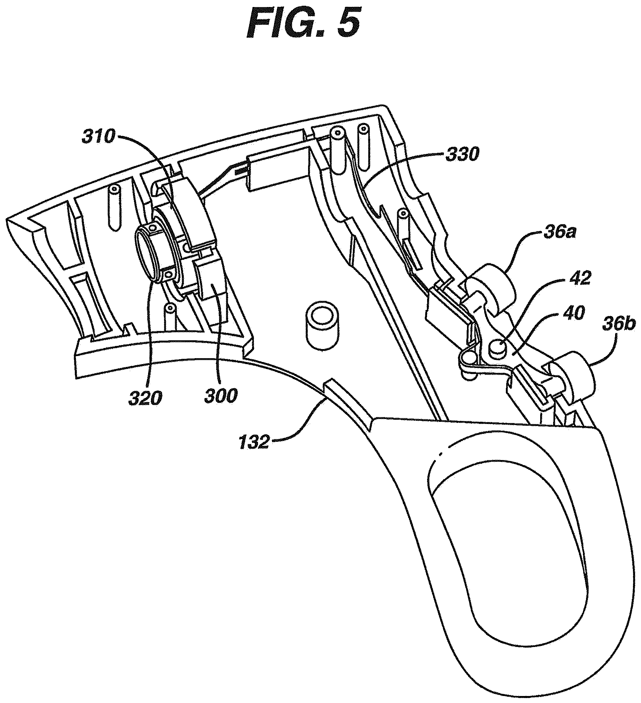

The housing 52 is preferably constructed from a aluminum, however, it is also contemplated that housing 52 may be made from a variety of plastics, such as Ultem ATM. A suitable ultrasonic drive unit 50 is model no. HP054, available from Ethicon Endo-Surgery, Inc., Cincinnati, Ohio. Two gold-plated circumferential electrical connectors 111a and 111b are located at the distal end of drive unit 50 for communicating electrical control signals from switches 36a-b to the generator 30. As shown in FIGS. 3 and 5, the handpiece 50 is preferably acoustically coupled to the second acoustic portion of the ultrasonic clamp coagulator apparatus 120. The distal end of the drive unit 50 is preferably coupled to the proximal end of the second acoustic portion by an internal threaded connection near an anti-node, but alternative coupling arrangements can be employed. When drive unit 50 is inserted into housing 130 and connected thereto, the distal end of drive unit 50 passes through connector 300 and ring connectors 111a-b interface with slip ring connectors 310 and 320, respectively, as is discussed in more detail below.

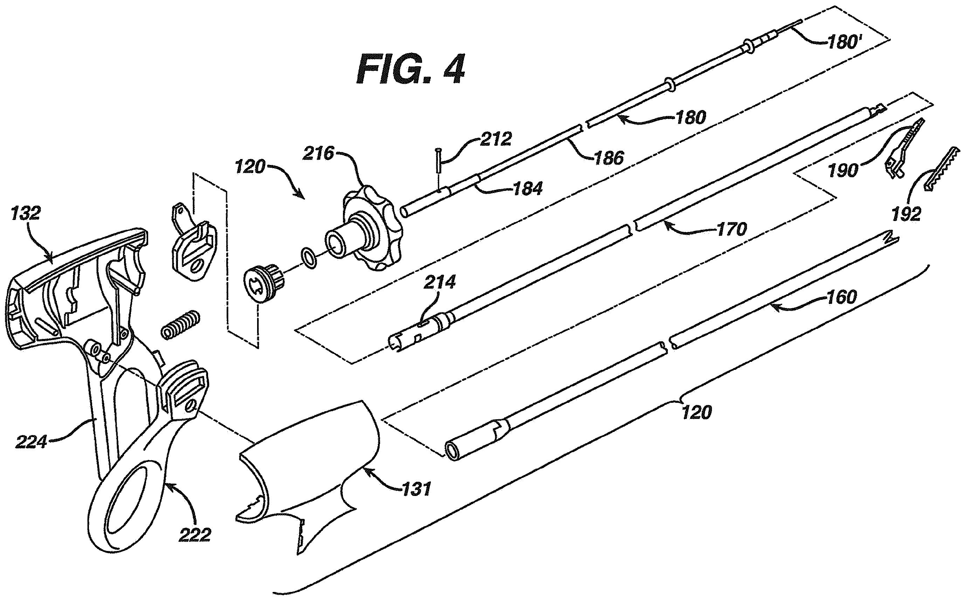

Referring also now to FIG. 4, an exploded view of the ultrasonic clamp coagulator apparatus 120 of the surgical system 10 in accordance with a preferred embodiment is illustrated. The proximal end of the ultrasonic clamp coagulator apparatus 120 preferably receives and is fitted to the distal end of the ultrasonic drive unit 50 by insertion of the drive unit into the housing of the apparatus, as shown in FIG. 3. The ultrasonic clamp coagulator apparatus 120 is preferably attached to and removed from the ultrasonic drive unit 50 as a unit. The ultrasonic clamp coagulator 120 may be disposed of after a single use.

The ultrasonic clamp coagulator apparatus 120 preferably includes a handle assembly or a housing 130, preferably comprising mating housing portions 131, 132, and an elongated or endoscopic portion 150. When the present apparatus is configured for endoscopic use, the construction can be 5 dimensioned such that portion 150 has an outside diameter of about 5.5 mm. The elongated portion 150 of the ultrasonic clamp coagulator apparatus 120 extends orthogonally from the apparatus housing 130. The elongated portion 150 can be selectively rotated with respect to the housing 130. The elongated portion 150 preferably includes an outer tubular member or sheath 160, an inner tubular actuating member 170, and the second acoustic portion of the acoustic system in the form of a waveguide 180 having an end-effector 180'. Outer tube 160, inner tube 170, end effector 180' and clamp pad 190 are all operatively coupled with rotation knob 216 so that rotation of knob 216 causes corresponding rotation of the end effector 180' and clamp arm 190. As illustrated in FIG. 4, the proximal end of the waveguide 180 of the second acoustic portion is preferably detachably coupled to the mounting device 84 of the ultrasonic drive unit 50 near an anti-node as described above. The waveguide 180 preferably has a length substantially equal to an integer number of one-half system wavelengths. The waveguide 180 is preferably fabricated from a solid core shaft constructed out of material that propagates ultrasonic energy efficiently, such as titanium alloy (i.e., Ti-6AI-4V) or an aluminum alloy. It is contemplated that the waveguide 180 can alternatively be fabricated from any other suitable material.

With particular reference to FIG. 2, a clamping mechanism of the present clamp coagulator 120 is configured for cooperative action with the end-effector 180' of the waveguide 180. The clamping mechanism includes a pivotally movable clamp arm 190, which is pivotally connected at the distal end thereof to the distal end of outer tubular sheath 160. A clamp pad 192, preferably formed from Teflon or other suitable low-friction material, is mounted on the surface of the clamp arm for cooperation with the end-effector 180', with pivotal movement of the clamp arm positioning the clamp pad in substantially parallel relationship to, and in contact with, the end-effector 180'. By this construction, tissue is grasped between the pad 192 and the end effector 180'. As illustrated, the pad 192 is preferably provided with a saw tooth-like configuration to enhance the gripping of tissue in cooperation with the end-effector 180'. As will be appreciated by those skilled in the art, end-effector 180' and clamp pad 190 may take on any number of shapes, including a curved shaped as disclosed in U.S. Patent No. 6,325,811.

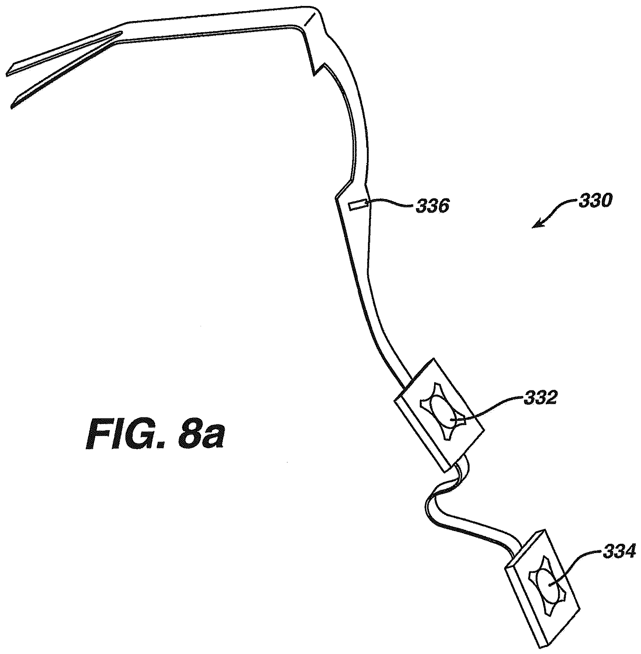

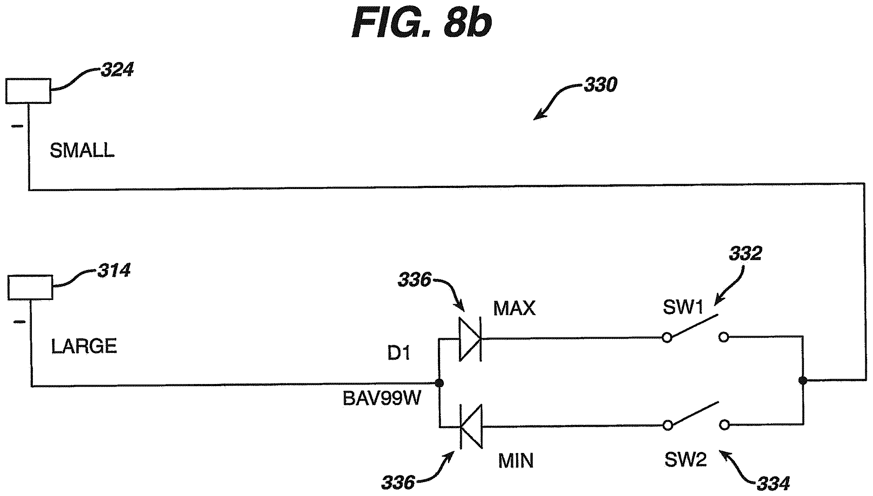

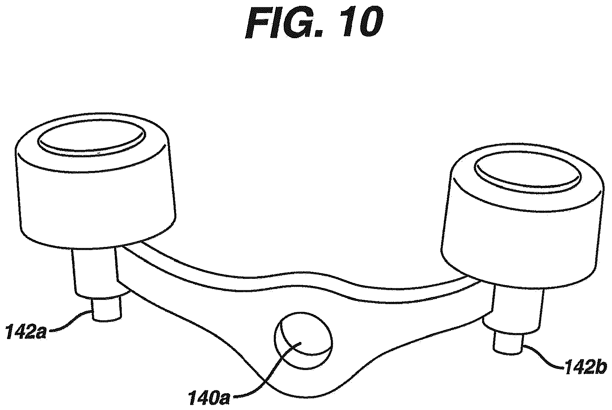

As is described in the U.S. patents previously mentioned, the surgeon's thumb squeezes trigger 222 to cause the clamping mechanism to pivot the movable clamp arm 190. One or more of the surgeon's other fingers may rest comfortably within handle 224. In accordance with the current invention, the surgeon's index finger controls the operation of the generator 30 by selectively depressing switches 36a-b. Switches 36a-b are conveniently located such that the surgeon may energize end effector 180' and also cause rotation of the end effector 180' and clamp pad 190 via knob 216 using the same hand (fingers) for operation. Referring now to Figs. 5-8 and 10, switches 36a-b are mechanically connected via a rocker arm 40 comprising an aperture 140a for accepting pivot post 42. In this configuration, switches 36a-b cannot be simultaneously depressed, which, if were the case, would provide an error message from generator 30. A flex circuit 330 provides for the electro-mechanical interface between switches 36a-b and the generator 30 via the drive unit 50. Also referring to Fig. 8a, flex circuit 330 includes, at the distal end, two dome switches 332 and 334 that are mechanically actuated by depressing pins 142a-b of corresponding switches 36a-b, respectively. Dome switches 332 and 334 are electrical contact switches, that when depressed provide an electrical signal to generator 30 as shown by the electrical wiring schematic of Fig. 8b. Flex circuit 330 also comprises two diodes within a diode package 336, also illustrated in Fig. 8b. Flex circuit 330 provides conductors, as is known to those in the art, that connect to slip ring conductors 310 and 320 via connector 300, which in turn provide electrical contact to ring conductors 111a-b, which in turn are connected to conductors in cable 32 that connect to generator 30. Ring conductors 111a-b are situated within the distal end of handpiece 50 as is generally described in U.S.

Patent No. 6,623,500 B1.

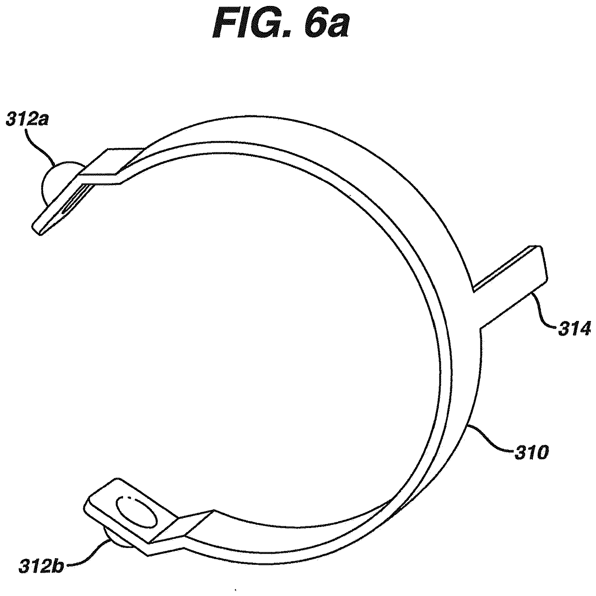

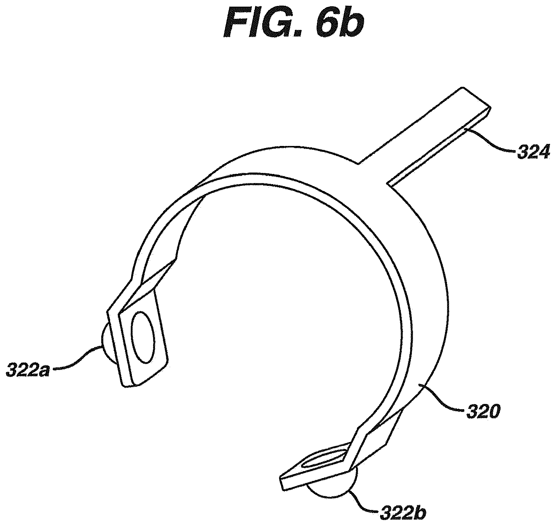

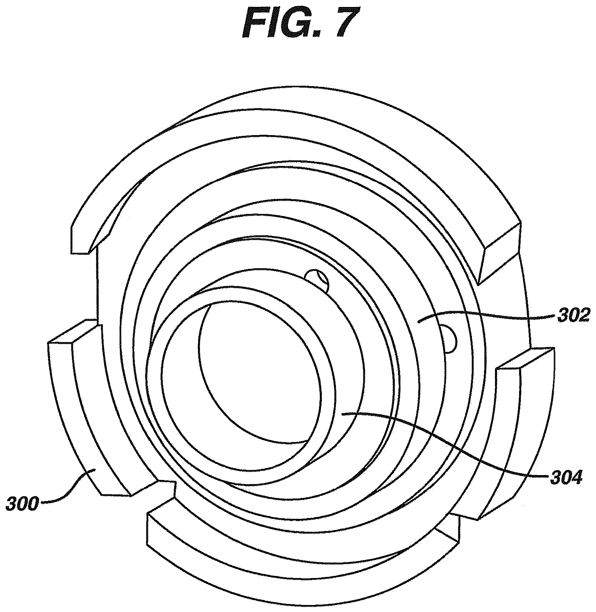

With particular reference now to Figs. 6a-b and 7, slip ring conductors 310 and 320 are generally open-ended O-shaped springs that slip onto mounting surfaces 302 and 304 of connector 300, respectively. Each spring slip-ring comprises two pressure point contacts (312a-b and 322a-b) that contact the respective ring conductor 111a-b of handpiece 50. The spring tension of the slip rings 310 and 320 cause positive contact between contacts 312a-b, 322a-b and conductors 111 a-b. It is evident that the slip-ring construction allows electrical contact to be made even as hand piece 50 may be rotated by the surgeon during use of the instrument. Posts 314 and 324 of the respective slip rings electrically connect to the respective conductor within flex circuit 330 to complete the electrical circuit as shown in Fig. 8b.

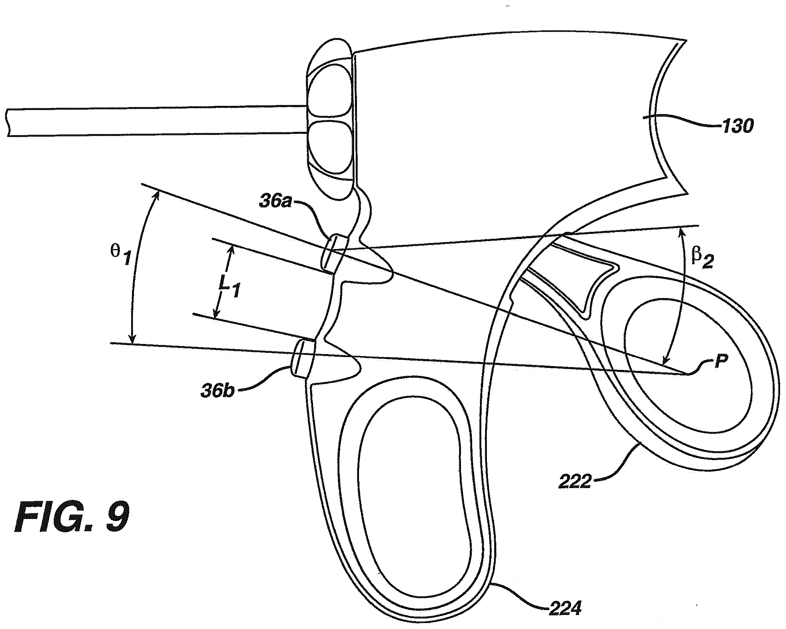

Referring now to Fig. 9, switches 36a-b are preferably configured in such a way to provide an ergonomically pleasing grip and operation for the surgeon. In particular, the angle of depression/activation of switches 36a-b is not parallel, but the direction of activation for each switch define an angle of actuation 81 with respect to a common point P within area of thumb placement of the thumb grip of trigger 222, when the trigger 222 is in its normal state. The range of angle 81 is from about 10° to about 30°, and more preferably from about 15° to about 20°. Switches 36a-b are also separated by a distance Li, which is sufficient to minimize inadvertent activation by the surgeon's finger resting on handle 224 between switches 36a-b, but at the same time provides for a high degree of grip stability during tissue grasping and manipulation functions. Distance L1 is from about 1 inch to about 0.5 inches, and more preferably, from about 0.8 inches to about 0.6 inches.

While the present invention has been illustrated by description of several embodiments, it is not the intention of the applicant to restrict or limit the scope of the appended claims to such detail. Numerous variations, changes, and substitutions will occur to those skilled in the art without departing from the scope of the invention. Moreover, the structure of each element associated with the present invention can be alternatively described as a means for providing the function performed by the element. Accordingly, it is intended that the invention be limited only by the scope of the appended claims.

## Claims

1. A housing (130) for an ultrasonic surgical instrument having a clamping mechanism configured for cooperative action with an end effector, the housing adapted to accept a handpiece (50) for providing ultrasonic energy, the housing comprising:
a handle (224) configured to interface with a user of the instrument;
a trigger (222) adapted to cause a clamp arm (190) of the clamping mechanism to pivot between a first position and a second position;
a rotation knob (216) adapted to cause corresponding rotation of the end effector and clamp arm;
**characterised in that** the housing further comprises at least one switch (36a-b) located on the handle and electrically connected to a generator (30) for providing an electrical signal to the generator (30) for controlling the level of ultrasonic energy delivered by the handpiece (50), the at least one switch located such that a user may operate the at least one switch and the rotation knob using the same finger(s) on the same hand interfacing with the handle;
a connector, wherein the connector (300) comprises at least one slip ring conductor (310, 320); and
a flex circuit (330) adapted to provide an electro-mechanical interface between the at least one switch (36a-b) and the generator (30), said flex circuit (330) comprising:
at least one electrical contact switch (332, 334) configured to provide, when depressed, an electrical signal to the generator (30); and
at least one conductor (111a-b) adapted to connect to a corresponding one of said at least one slip ring conductors (310, 320) via said connector (300).

2. The housing (130) of claim 1, wherein the handle comprises a pistol-type grip configuration.

3. The housing (130) of claim 1 or claim 2, comprising two switches (36a-b), the first switch electrically connected to the generator (30) for providing a first electrical signal to the generator (30), and the second switch electrically connected to the generator (30) for providing a second electrical signal to the generator (30).

4. The housing (130) of any preceding claim, wherein the handpiece (50) comprises an electrical conductor for accepting the electrical signal from the switch.

5. The housing (130) of claim 3, wherein the two switches (36a-b) are separated by a distance from about one-half inch to about one inch.

6. The housing (130) of claim 3, wherein the first and second switches (36a-b) each define a line of actuation that form an angle of actuation from about 10° to about 30°.

7. The housing (130) of any preceding claim, further comprising a proximal opening for accepting said handpiece (50) and a distal opening for accepting an ultrasonic waveguide, wherein the proximal opening and distal opening define a longitudinal axis.

8. The housing (130) of any of claims 1 and 3 to 7, wherein the first switch provides for a first level of amplitude and the second switch provides for a second level of amplitude.

## Patentansprüche

1. Gehäuse (130) für ein chirurgisches Ultraschallinstrument mit einem Klemmmechanismus, der zum Zusammenwirken mit einem Endeffektor ausgelegt ist, wobei das Gehäuse dazu angepasst ist, ein Handstück (50) zum Bereitstellen von Ultraschallenergie aufzunehmen, wobei das Gehäuse umfasst:
einen Griff (224), der dazu ausgelegt ist, eine Schnittstelle zu einem Benutzer des Instruments zu bilden;
einen Auslöser (222), der dazu angepasst ist, zu bewirken, dass ein Klemmarm (190) des Klemmmechanismus zwischen einer ersten Position und einer zweiten Position schwenkt;
einen Drehknopf (216), der dazu angepasst ist, eine entsprechende Drehung des Endeffektors und des Klemmarms zu bewirken;
**dadurch gekennzeichnet, dass** das Gehäuse ferner mindestens einen Schalter (36a-b) umfasst, der an dem Schalter angeordnet und elektrisch mit einem Generator (30) verbunden ist, zum Bereitstellen eines elektrischen Signals für den Generator (30) zum Steuern des Niveaus der von dem Handstück (50) gelieferten Ultraschallenergie, der mindestens eine Schalter derart angeordnet ist, dass ein Benutzer den mindestens einen Schalter und den Drehknopf mit den Fingern der Hand bedienen kann, die mit dem Griff zusammenwirkt;
einen Verbinder, wobei der Verbinder (300) mindestens einen Schleifringleiter (310, 320) umfasst; und
eine flexible Schaltung (330), die dazu angepasst ist, eine elektromechanische Schnittstelle zwischen dem mindestens einen Schalter (36a-b) und dem Generator (30) bereitzustellen, wobei die flexible Schaltung (330) umfasst:
mindestens einen elektrischen Kontaktschalter (332, 334), der dazu ausgelegt ist, wenn er gedrückt wird, ein elektrisches Signal für den Generator (30) bereitzustellen; und
mindestens einen Leiter (111a-b), der dazu angepasst ist, mit einem entsprechenden einen des mindestens einen Schleifringleiters (310, 320) über den Verbinder (300) verbunden zu sein.

2. Gehäuse (130) nach Anspruch 1, wobei der Griff eine pistolenartige Griffauslegung umfasst.

3. Gehäuse (130) nach Anspruch 1 oder Anspruch 2, umfassend zwei Schalter (36a-b), wobei der erste Schalter elektrisch mit dem Generator (30) verbunden ist, zum Bereitstellen eines ersten elektrischen Signals für den Generator (30), und wobei der zweite Schalter elektrisch mit dem Generator (30) verbunden ist, zum Bereitstellen eines zweiten elektrischen Signals für den Generator (30).

4. Gehäuse (130) nach einem vorhergehenden Anspruch, wobei das Handstück (50) einen elektrischen Leiter zum Annehmen des elektrischen Signals von dem Schalter umfasst.

5. Gehäuse (130) nach Anspruch 3, wobei die zwei Schalter (36a-b) durch einen Abstand von ungefähr einem halben Zoll bis ungefähr einem Zoll (ungefähr 1,27 cm bis ungefähr 2,54 cm) getrennt sind.

6. Gehäuse (130) nach Anspruch 3, wobei der erste und der zweite Schalter (36a-b) jeweils eine Betätigungsleitung definieren, die einen Betätigungswinkel von ungefähr 10° bis ungefähr 30° bilden.

7. Gehäuse (130) nach einem vorhergehenden Anspruch, ferner umfassend eine proximale Öffnung zum Aufnehmen des Handstücks (50) und eine distale Öffnung zum Aufnehmen eines Ultraschallwellenleiters, wobei die proximale Öffnung und die distale Öffnung eine Längsachse definieren.

8. Gehäuse (130) nach einem der Ansprüche 1 und 3 bis 7, wobei der erste Schalter ein erstes Niveau der Amplitude bereitstellt und der zweite Schalter ein zweites Niveau der Amplitude bereitstellt.

## Revendications

1. Boîtier (130) pour un instrument chirurgical ultrasonore possédant un mécanisme de serrage conçu pour une action coopérative avec un effecteur terminal, le boîtier étant conçu pour accepter une pièce à main (50) pour fournir une énergie ultrasonore, le boîtier comprenant :
une poignée (224) conçue pour assurer l'interface avec un utilisateur de l'instrument ;
une détente (222) conçue pour amener un bras de serrage (190) du mécanisme de serrage à pivoter entre une première position et une seconde position ;
un bouton de rotation (216) conçu pour provoquer une rotation correspondante de l'effecteur terminal et du bras de serrage ;
**caractérisé en ce que** le boîtier comprend en outre au moins un commutateur (36a-b) situé sur la poignée et connecté électriquement à un générateur (30) pour fournir un signal électrique au générateur (30) pour commander le niveau d'énergie ultrasonore délivrée par la pièce à main (50), l'au moins un commutateur étant situé de sorte qu'un utilisateur puisse faire fonctionner l'au moins un commutateur et le bouton de rotation à l'aide du ou des mêmes doigts de la main assurant l'interface avec la poignée ;
un connecteur, le connecteur (300) comprenant au moins un conducteur annulaire coulissant (310, 320) ; et
un circuit souple (330) conçu pour assurer une interface électromécanique entre l'au moins un commutateur (36a-b) et le générateur (30), ledit circuit souple (330) comprenant :
au moins un commutateur de contact électrique (332, 334) configuré pour fournir, lorsqu'il est appuyé, un signal électrique au générateur (30) ; et
au moins un conducteur (111a-b) conçu pour se connecter à un conducteur correspondant dudit ou desdits conducteurs annulaires coulissants (310, 320) par l'intermédiaire dudit connecteur (300).

2. Boîtier (130) selon la revendication 1, dans lequel la poignée comprend une configuration de préhension de type pistolet.

3. Boîtier (130) selon la revendication 1 ou la revendication 2, comprenant deux commutateurs (36a-b), le premier commutateur étant connecté électriquement au générateur (30) pour fournir un premier signal électrique au générateur (30), et le second commutateur étant connecté électriquement au générateur (30) pour fournir un second signal électrique au générateur (30).

4. Boîtier (130) selon l'une quelconque des revendications précédentes, dans lequel la pièce à main (50) comprend un conducteur électrique pour accepter le signal électrique provenant du commutateur.

5. Boîtier (130) selon la revendication 3, dans lequel les deux commutateurs (36a-b) sont séparés par une distance d'environ un demi-pouce à environ un pouce.

6. Boîtier (130) selon la revendication 3, dans lequel les premier et second commutateurs (36a-b) définissent chacun une ligne d'actionnement formant un angle d'actionnement d'environ 10° à environ 30°.

7. Boîtier (130) selon l'une quelconque des revendications précédentes, comprenant en outre une ouverture proximale pour accepter ladite pièce à main (50) et une ouverture distale pour accepter un guide d'onde ultrasonore, dans lequel l'ouverture proximale et l'ouverture distale définissent un axe longitudinal.

8. Boîtier (130) selon l'une quelconque des revendications 1 et 3 à 7, dans lequel le premier commutateur fournit un premier niveau d'amplitude et le second commutateur fournit un second niveau d'amplitude.
